# EUROPEAN PATENT APPLICATION

(11) **EP 1 447 453 A1**
(43) Date of publication of application: **18.08.2004**
(21) Application number: 03003133.0
(22) Date of filing: 13.02.2003
(51) Int. Cl.: C12Q 1/68, C12N 15/09, C12N 15/63

(54) **System for inducible, local and reversible gene silencing using RNA interference**

(71) Applicant: MAX-PLANCK-GESELLSCHAFT ZUR FÖRDERUNG DER WISSENSCHAFTEN E.V., 14195 Berlin (DE)
(72) Inventor: Lutz, Beat, 80807 München (DE); Brenz Verca, Maria, 85521 Ottobrunn (DE)
(74) Representative: VOSSIUS & PARTNER

(57) **Abstract**

The present invention relates to a combination of expression cassettes for interfering with the expression of at least one gene in a cell or a vertebrate comprising (a) an expression cassette comprising a first promoter and operatively linked thereto a polynucleotide encoding an inducible transactivator (ITA); (b) one or more expression cassettes comprising polynucleotide(s) encoding one or more trans-acting factors (TAFs) heterologous to said cell or vertebrate and necessary and sufficient to functionally interact with a pol I promoter wherein expression of at least one of said TAFs is directed by a second promoter inducible by the transactivator; and (c) at least one expression cassette comprising at least one pol I promoter and operatively linked thereto one or more polynucleotides giving rise upon transcription and optionally post-transcriptional modifications RNA molecules capable of interfering with gene expression.

## Description

The present invention relates to a combination of expression cassettes for interfering with the expression of at least one gene in a cell or a vertebrate comprising (a) an expression cassette comprising a first promoter and operatively linked thereto a polynucleotide encoding an inducible transactivator (ITA); (b) one or more expression cassettes comprising (a) polynucleotide(s) encoding one or more trans-acting factors (TAFs) heterologous to said cell or vertebrate and necessary and sufficient to functionally interact with a pol I promoter wherein expression of at least one of said TAFs is directed by a second promoter inducible by the transactivator; and (c) at least one expression cassette comprising at least one pol I promoter and operatively linked thereto one or more polynucleotides giving rise upon transcription and optionally post-transcriptional modifications RNA molecules capable of affecting gene expression.

In the specification a number of documents is cited. The disclosure content of these documents including manufacturer's instructions, is herewith incorporated by reference.

Genetic approaches of silencing gene function in a transient and region-specific manner, e.g. in the brain, have been recently under intensive investigations. The method of conditional gene targeting using the Cre/loxP system enables to induce gene deficiencies in a spatiotemporal manner, but these changes are irreversible (Rajewsky et al., 1996; Kwan, 2002). Alternatively, the method of tetracycline-inducible and region-specific overexpression of gene constructs that encode e.g. dominant-negative forms of proteins (Malleret et al., 2001) has also been applied very successfully in mice. However, this method relies on the availability of dominant-negative forms of the targeted proteins, which can constitute a major restraint. In addition, overexpression of such proteins might induce unpredictable effects that impede the interpretation of the induced phenotype.

RNA interference (RNAi) represents a regulatory biological response to exogenously introduced double stranded RNA (dsRNA) causing sequence-specific mRNA degradation. dsRNAs of 50 to 500 bp in length are normally used in *C. elegans* and *Drosophila* as a reverse genetic tool to inhibit the expression of RNA of interest. However, such long dsRNAs cause unspecific responses in mammalian cells leading to general inhibition of protein expression. In 2001, Tuschl and co-workers discovered that it is possible to specifically destroy the mRNA of interest by applying duplexes of 21-nt RNAs to cultured mammalian cells. The delivery of siRNA was able to elicit robust and sequence specific silencing effects (Elbashir et al., 2001).

Whereas RNAi is mediated very efficiently by siRNA in mammalian cell culture systems, it does not allow an inducible and heritable expression, and consequently, it is not suitable for the generation of stable cell lines and transgenic mice or rats. Generally, expression vectors are based on polymerase II (pol II)-dependent promoter systems. Pol II synthesizes all protein-encoding mRNAs, which undergo extensive modifications after synthesis by addition of 5' cap nucleotides and polyA tails. These modifications, however, block the RNAi effect, making therefore pol II-based vectors unapplicable for the expression of siRNA.

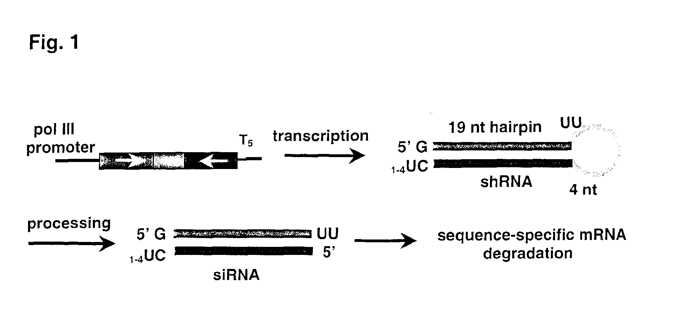

In early 2002, a major advance was achieved by generating polymerase III (pol III)-based vectors for expression of short 21-nt RNA molecules (Fig. 1). Some of these vectors have been engineered to express short hairpin RNAs (shRNAs), which get processed in vivo into siRNA molecules capable of carrying out gene-specific silencing (e.g. Brummelkamp et al., 2002; Paddison et al., 2002; Paul et al., 2002; Yu et al., 2002). These vectors contain the short hairpin sequence between a pol III promoter and a transcription termination site of 4-5 thymidine residues. The transcript is terminated at position 1-4 of the termination site (pol III transcripts naturally lack polyA tails) and then folds into a hairpin structure with 3' ₁₋₄U-overhangs. The loop end of the shRNAs are processed in vivo, generating 21 nt siRNA molecules, which in turn initiate RNAi. This processing is reminicent to the processing of shRNAs naturally occurring in *C. elegans* (Lee & Ambros, 2001).

Another siRNA expression vector developed by a different research group encodes the sense and antisense siRNA strands under control of two separate pol III promoters (Miyagishi et al., 2002). The siRNA strands from this vector, like the shRNAs of the other vectors, have termination signals of 5 thymidine residues. Silencing efficacy by both types of expression vectors is comparable or even better to that induced by transiently transfected siRNA.

All pol III-based expression system published to date are suitable for establishing stable cell lines. However, the ideal system for gene silencing should also include the possibility of regulation, both in terms of temporal and spatial resolution. Concerning the temporal resolution, a tetracycline-based inducible system was applied to the pol III-driven promoter of the U6 snRNA gene (Ohkawa & Taira, 2000). In this system, two tetracycline operator (TetO) sequences were introduced between the proximal promoter sequence (the binding site for snRNA-activating protein complex SNAPc) and the TATA-box (the binding site for TATA box-binding protein TBP). Tetracycline repressor (TetR), expressed from an independent vector, binds to TetO, thus providing a positional interference with the transcription machinery by blocking the transcription factor IIIB-mediated bridging between SNAPs and TBP. Addition of tetracycline to the cells induces the release of TetR from TetO and, thereby, it induces pol III-driven transcription. However, this system lacks spatial resolution. As pol III promoters are active ubiquitously, TetR must be expressed also ubiquitously to allow the repression of transcription. Such a system will certainly be very useful for a number of applications where only temporal but not spatial resolution is required. Genetic analyses of e.g. adult brain function, however, desires this spatial resolution, thus, this inducible pol III-driven expression system must be modified for its potential use in transgenic mammals. Thus, the technical problem underlying the present invention was to provide a system that reliably allows silencing gene function in a transient and region-, cell-, or tissue-specific manner. The solution to this technical problem is achieved by providing the embodiments characterized in the claims.

Accordingly, the present invention relates to a combination of expression cassettes for interfering with the expression of at least one gene in a cell or a vertebrate comprising
(a) an expression cassette comprising a first promoter and operatively linked thereto a polynucleotide encoding an inducible transactivator (ITA);
(b) one or more expression cassettes comprising (a) polynucleotide(s) encoding one or more trans-acting factors (TAFs) heterologous to said cell or vertebrate and necessary and sufficient to functionally interact with a pol I promoter wherein expression of at least one of said TAFs is directed by a second promoter inducible by the transactivator; and
(c) at least one expression cassette comprising at least one pol I promoter and operatively linked thereto one or more polynucleotides giving rise upon transcription and optionally post-transcriptional modifications RNA molecules capable of affecting gene expression.

In accordance with the present invention, the term "expression cassette" denotes a cloning vehicle or vector which is constructed at least partially in a modular way such that elements contained in the cloning vehicle/vector can easily be replaced by different elements. Such elements may be promotors or other regulatory elements, coding regions, e.g. for antibiotics etc.

A "combination of expression cassettes" refers to a variety of different expression cassettes wherein the variety, in accordance with the invention, comprises at least three different expression cassettes. The expression cassettes may be present as distinct entities. Distinct entities refers to the fact that the expression cassettes of the invention are physically distinct molecular entities i.e. different vectors that are separately propagated. Alternatively, at least two of the expressions cassettes may be comprised on the same vector and may thus be propagated simultaneously.

As stated above, the expression cassettes may be part of an expression vector. Said vector may be, for example, a phage, plasmid or viral vector. Retroviral vectors may be replication competent or replication defective. In the latter case, viral propagation generally will occur only in complementing host/cells.

The polynucleotides or genes of the invention may be joined to a vector containing selectable markers for propagation in a host. Generally, a plasmid vector is introduced in a precipitate such as a calcium phosphate precipitate or rubidium chloride precipitate in a complex with a charged lipid or by electroporation. Should the vector be a virus, it may be packaged in vitro using an appropriate packaging cell line prior to application to host cells.

In the expression cassettes comprised in the vector, the polynucleotide is operatively linked to expression control sequences allowing expression in prokaryotic or eukaryotic cells or isolated fractions thereof.
Expression of said polynucleotide comprises transcription of the polynucleotide, where required into a translatable mRNA. Regulatory elements ensuring expression in eukaryotic cells, preferably mammalian cells, are well known to those skilled in the art. They comprise the above recited promoter sequences ensuring initiation of transcription and optionally, where desired, poly-A signals ensuring termination of transcription and stabilization of the transcript. Additional regulatory elements may include transcriptional as well as translational enhancers. Possible regulatory elements permitting expression in prokaryotic host cells comprise, e.g., the *lac, trp* or *tac* promoter in *E*. *coli,* and examples for regulatory elements permitting expression in eukaryotic host cells are the *AOX1* or *GAL 1* promoter in yeast or the CMV-, SV40-, RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. In this context, suitable expression vectors which may form a basis for the vectors to be used in accordance with the invention upon appropriate modification are known in the art such as Okayama-Berg cDNA expression vector pcDV1 (Pharmacia), pCDM8, pRc/CMV, pcDNA1, pcDNA3 (Invitrogene), pSPORT1 (GIBCO BRL). Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the polynucleotides or vector into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (2001). Alternatively, the polynucleotides and vectors of the invention can be reconstituted into liposomes for delivery to target cells.

The term "interfering with the expression of at least one gene" means, in accordance with the present invention, that the expression of the gene is reduced by at least 30%, preferably at least 50%, more preferred at least 75%, even more preferred at least 90% and most preferred at least 95% such as at least 98% or even at least 99%. Optionally, expression is completely shut down. In this case, expression is reduced essentially to 100%.

The term "transactivator" denotes, in accordance with the present invention, a molecule, preferably a (poly)peptide that activates expression of a (poly)peptide encoded by a DNA sequence contained in an expression cassette but that is itself not encoded by said expression cassette. Said transactivator is "inducible" in accordance with the present invention, i.e. the transactivator is not normally functional but may be induced upon the addition of a suitable inducer. As is well known in the art, there are a variety of inducers that act on transactivators such as provided in examples in claims 9 and 10. Further examples of inducers are mentioned herein below.

The term "trans-acting factor" means, in accordance with the invention, a heterologous to host cell or animal molecule, preferably a (poly)peptide that activates expression of a (poly)peptide encoded by a DNA sequence contained in an expression cassette but that is itself not encoded by said expression cassette. Said DNA sequence is preceded by heterologous to host cell or animal polymerase I promoter coming from the same species as said trans-acting factor.

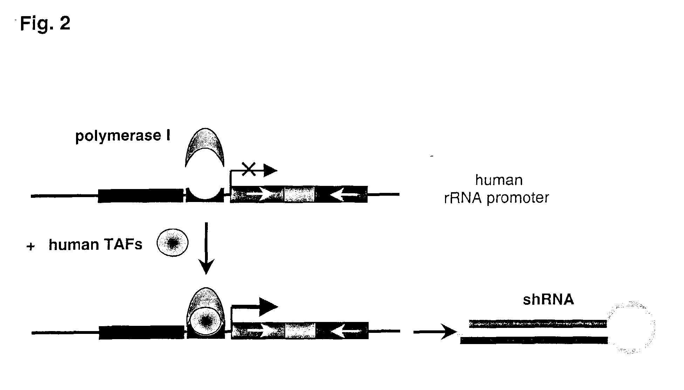

To overcome the technical problem outlined above, a fully novel, heritable and regulatable system of gene silencing using polymerase I (pol I)-based vectors was developed. Pol I normally transcribes the majority of ribosomal RNA (rRNA) genes in the cells. Pol I shares several advantages with pol III as compared to pol II in terms of absence of post-transcriptional RNA modifications (i.e. lack of capping and polyadenylation). Extensive characterization of the rRNA transcription machinery allows the design of rRNA-based vectors, expressing RNA of precise length and sequence as required by the experimental needs. In addition, pol I-dependent transcription shows remarkable species specificity, which is very unusual as compared to pol II and pol III. For example, human rRNA genes are completely silent in mouse cell extracts, and the opposite is also true (Heix & Grummt, 1995). At a particular site of the mouse pol I promoter, one or several human trans-acting factors (TAFs) mediate this species specificity. In accordance with the invention, expression of at least one of these TAFs depends on the expression of the transactivator. For the TAFs' capability to functionally interact with the pol I polymerase promoter, it is necessary that the promoter and the TAFs originate from the same species. It follows or is required that the pol I polymerase promoter is heterologous with respect to the cell or vertebrate. The rest of the functional units of the promoter as well as the majority of transcription machinery components including pol I itself are functionally interchangeable between the two species. Consequently, the activity of the human promoter could be restored by introducing human trans-acting factors into mouse cells, by expressing them from an independent pol II promoter (Fig. 2).

One important property of this strategy is the fact that the basic construct (exemplified here with regard to mouse and human, but applicable to other species as well) with the human pol I promoter is silent on its own in mouse, and becomes active only upon addition of the human TAFs. This allows the construction of a pol I-based promoter, which can be regulated in both a time- and tissue-specific manner by expressing human TAFs from inducible and tissue-specific promoters (see below).

The same vector as one described above (Fig. 2) can be used in accordance with the present invention to inhibit the expression of several genes simultaneously. In this case two or more shRNAs-coding fragments may be cloned in a e.g. tail-to-head configuration into the same vector under the control of an inducible pol I promoter. The strategy allows a convenient method to simultaneously target either several members of the same gene family or completely unrelated genes, including easily detectable reporter markers. This system represents a considerable advantage to the technically demanding knock-out technologies, where every gene must be targeted independently.

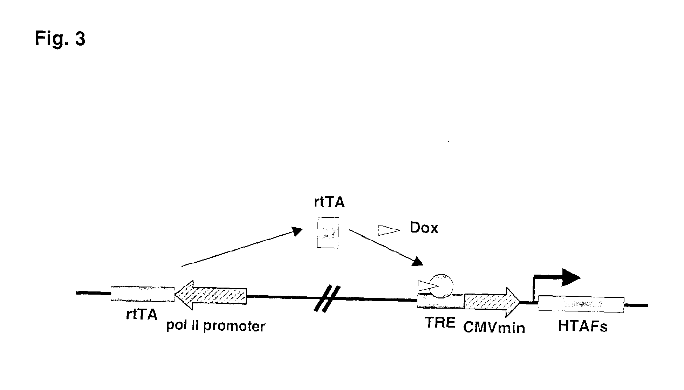

The strategy devised in accordance with the invention allows to construct vectors that enable to express e.g. human TAFs in an inducible and reversible manner. In Fig. 3 this principle is shown for a preferred embodiment of the invention wherein the inducer is doxycycline.

The combination of expression cassettes developed in accordance with the present invention can be easily combined with existing viral vectors to facilitate its delivery into model systems difficult to be transfected, such as primary neuronal or organotypic brain cultures, or intact brain. In order to perform RNA interference in the mouse, two transgenic mouse lines have to be established, one expressing human TAFs in a tissue-specific and inducible manner (construct in Fig. 3), and second expressing one or several shRNAs under inducible pol I promoter (construct in Fig. 2).

The present invention thus provides a unique system that allows the cell- or tissue specific interference with gene expression in a time-dependant manner. In particular, the following advantages are associated with the combination of expression cassettes designed for said interference:

High versatility, provided by combination of features such as time- and region- (cell-, tissue- etc.) specificity, as well as reversibility of gene silencing.

The possibility to study the function of essential genes by inhibiting their expression only at certain time in certain tissues, thus avoiding the interference with organism's survival and basic vital functions.

The possibility to inhibit several genes simultaneously or independently. In the first case, several shRNAs are expressed as a cluster of stem-loop structures under the control of the same pol I promoter. In the second case, several genes are targeted by shRNAs transcribed from pol I and pol III-driven expression cassettes, respectively. Thus, two promoters can be regulated in an independent fashion, and competition at the level of transcription factors is avoided.

High flexibility of targeting choices, ranging from one specific splice variant of a particular gene to an entire gene family of interest.

Usefulness of the system in several models to study gene function. It can be applied locally to cell cultures, tissue cultures or intact organs by using chemical means of delivery or viral vectors for DNA application or it can be expressed in a stable fashion both in cell cultures or in non-human transgenic mammals.

In a preferred embodiment of the combination of expression cassettes of the invention, said RNA molecules are short interfering RNAs (siRNAs) having a region of identity of 19 to 29 nucleotides of at least 90% with the exonic sequences of at least one target gene, ribozymes, antisense RNA oligonucleotides, RNA decoys, triplex-forming RNA oligonucleotides or RNA aptamers.
It is further preferred in accordance with this embodiment that the sequence of identity is at least 95% such as at least 98%, 99% or even 100%. Further preferred is that the overall length of the siRNA does not exceed 29 nucleotides. It is additionally preferred that the sequence of identity is between 19 and 23 such as 20, 21, or 22 nucleotides and that the overall length of the siRNA molecule does not exceed the number of identical nucleotides (in this type of embodiment, the sequence of identity may range between 90 and 100% and assume the values that have been pointed out above with the most preferred option being an identity value of 100%). The sequence of identity may be flanked by sequences of non-identity but this option is not preferred. Whenever the sequence of identity is flanked by a sequence of non-identity, it is preferred that the overall length of the siRNA does not exceed about 45 to 50 nucleotides.

"Antisense RNA oligonucleotides" means RNA constructs which block the expression of the naturally occurring gene product.

In general, the mode of action of any of the above molecules interfering with gene expression is well known in the art. When using the antisense approach for reduction of the amount of proteins of interest in cells, the nucleic acid molecule encoding the antisense-RNA displays a high degree of homology to endogenously occurring nucleic acid molecules encoding said protein. In this case the homology is preferably higher than 80%, particularly higher than 90% and still more preferably higher than 95%.
In a further preferred embodiment of the combination of expression cassettes of the invention, said promoter inducible by said transactivator is a pol II polymerase promoter.

Particularly preferred is a combination of expression cassettes wherein said first promoter is a pol II polymerase promoter and is cell-type specific.
The term "a pol II polymerase promoter... is cell-type specific" means, as is generally recognized in the art, that the promoter will induce transcription in a certain type of cell but not in a different type of cell. Whether a promoter is active in a certain type of cell depends in this regard on the differentiation status of the cell. For example a promoter may be active in the pancreas but not in the heart. An example of such a promoter is the insulin promoter.

The vertebrate referred to herein may be any vertebrate such as a rodent, chicken, frog, fish. However, it is preferred that said vertebrate is a mammal.

In a most preferred embodiment of the combination of expression cassettes of the invention said mammal is a rodent. A prime example of a rodent wherein the combination of expression cassettes is employed is a mouse such as a mouse belonging to a laboratory strain, e.g. BALB/c, C57B16, CBA, DBA, AKR or Sv129 or a rat, also preferably belonging to a laboratory strain, e.g., Wistar, Fischer 344, Lewis. Rodents are, in particular due to the well established protocols for generating transgenic mice or rats, preferred as models for studying the interference with the expression of genes. These genes may be tissue-specific genes, developmental genes or genes engaged in differentiation processes, to name a few examples.

In an additional preferred embodiment of the combination of expression cassettes of the invention, said pol I polymerase promoter is a human pol I polymerase promoter.

Further preferred in accordance with the combination of expression cassettes of the invention is that said transactivator is inducible by an antibiotic or hormone.

It is particularly preferred that said antibiotic or hormone is tetracycline, doxycycline, ecdysone, ponasterone A, RU486, tamoxifen or a glucocorticoid hormone.

In another preferred embodiment of the combination of expression cassettes of the invention, said inducible transactivator is a tetracycline-dependent transactivator (tTA), a reverse tetracycline-dependent transactivator (rtTA), an ecdysone receptor, a gal4-PR progesterone receptor chimera, a gal4-ER estrogen receptor chimera or glucocorticoid receptor (GR).
In accordance with this embodiment, it is preferred that the TAF expression is under the direct control of a second promoter containing the corresponding ITA responsive elements.

Also preferred is a combination of expression cassettes further comprising at least one further polynucleotide molecule comprising a pol III promoter-driven expression cassette giving rise upon transcription and optionally posttranscriptional modifications to siRNA molecules having a region of identity of 19 to 29 nucleotides of at least 90% with the coding region of another target gene, or to ribozymes, antisense RNA oligonucleotides, RNA decoys, triplex-forming RNA oligonucleotides or RNA aptamers interfering with the expression of another target gene.
The advantages of including into the system interfering molecules the expression of which is driven by a pol III polymerase promoter have been referred to herein above. Briefly, two sets of interfering molecules can be expressed and regulated independently by pol I and pol III-driven expression cassettes that provides higher versatility to the system. In addition, the use of two different polymerases promoters excludes competition and mutual inhibition at the level of transcription machinery factors.

In a different preferred embodiment of the combination of expression cassettes according to the invention, the TAFs are TBP (TATA-binding protein), and TATA-associated factors: TAFₗ48, TAFₗ63 and TAFₗ110.

The present invention also relates to a method of interfering with the expression of at least one target gene in a cell or a vertebrate comprising the step of providing said cell or said vertebrate carrying the combination of expression cassettes of the invention, with an inducer of said transactivator.
The vertebrate that receives the inducer may be a transgenic animal that is the offspring of two transgenic animals carrying different transgenic constructs. A preferred example of such parental transgenic animals has been provided herein above. Namely, one parental transgenic animal may harbor a construct as shown in figure 2 whereas the second parent harbors the construct as shown in figure 3.

The inducer may be administered to the vertebrate in a variety of manners, depending on its nature and the experimental set-up, For example, if the inducer is an antibiotic, it may be provided as a food supplement. Alternatively, the inducer may be injected or applied topically. Other modes of administration are feasible.

Another subject of the invention is a non-human transgenic animal harbouring the combination of expression cassettes according to the invention.

A method for the production of a transgenic non-human animal, for example transgenic mouse, comprises introduction of the above expression cassettes or at least one of said expression cassettes optionally comprised in a targeting vector into a germ cell, an embryonic cell, stem cell or an egg or a cell derived therefrom. Production of transgenic embryos and screening of those can be performed, e.g., as described by A. L. Joyner Ed., Gene Targeting, A Practical Approach (1993), Oxford University Press. The DNA of the embryonal membranes of embryos can be analyzed using, e.g., Southern blots with an appropriate probe; see supra. A general method for making transgenic non-human animals is described in the art, see for example WO 94/24274. For making transgenic non-human organisms (which include homologously targeted non-human animals), embryonal stem cells (ES cells) are preferred. Murine ES cells, such as AB-1 line grown on mitotically inactive SNL76/7 cell feeder layers (McMahon and Bradley, Cell 62: 1073-1085 (1990)) essentially as described (Robertson, E. J. (1987) in Teratocarcinomas and Embryonic Stem Cells: A Practical Approach. E. J. Robertson, ed. (Oxford: IRL Press), p. 71-112) may be used. Other suitable ES lines include, but are not limited to, the E14 line (Hooper et al., Nature 326: 292-295 (1987)), the D3 line (Doetschman et al., J. Embryol. Exp. Morph. 87: 27-45 (1985)), the CCE line (Robertson et al., Nature 323: 445-448 (1986)), the AK-7 line (Zhuang et al., Cell 77: 875-884 (1994) which is incorporated by reference herein). The success of generating a mouse line from ES cells bearing a specific transgene depends on the pluripotence of the ES cells (i. e., their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant nonhuman females and are born as chimeric mice. The resultant transgenic mice are chimeric for cells having either the expression cassettes and are backcrossed and screened for the presence of the cassettes/transgene(s) by PCR or Southern blot analysis on tail biopsy DNA of offspring so as to identify transgenic mice heterozygous for either the recombinase or reporter locus/loci.
Methods for producing transgenic flies, such as Drosophila melanogaster are also described in the art, see for example US-A-4,670,388, Brand & Perrimon, Development (1993) 118: 401-415; and Phelps & Brand, Methods (April 1998) 14: 367-379.
Transgenic worms such as C. elegans can be generated as described in Mello, et al., (1991) Efficient gene transfer in C.elegans: extrachromosomal maintenance and integration of transforming sequences. EMBO J. 10, 3959-70, Plasterk, (1995) Reverse genetics: from gene sequence to mutant worm. Methods Cell Biol 48, 59-80.

The invention further relates to cell line harbouring the combination of expression cassettes of the invention.
A "cell line" is defined in the art as a culture of cells of defined origin that is capable of permanently growing in a culture medium. The cells of a cell line are normally uniform in their morphology and with regard to their functional characteristics. Many cell lines established in the art are of tumorous origin. The same definition of a cell line is applied here.
The cell line of the invention may be a eukaroytic cell line of various origins. Preferably, it is a cell line such as a mammalian, preferably human, or insect, yeast, fungal cell line.

Finally, the present invention relates to a kit of parts comprising the combination of expression cassettes of the invention. The kit is preferably used as a diagnostic composition. The various components of the kit may be packaged in one or more containers such as one or more vials. The vials may, in addition to the components comprise preservatives or buffers for storage.

The figures show:
**Figure 1:** Schematic representation of pol III-driven transcription of siRNA in mammalian cells, using the U6 snRNA promoter (Paul et al., 2002).
**Figure 2:** Schematic representation of the inducible pol I-based expression system for the generation of siRNAs. In the mouse, transcription cannot be initiated from the human (black box) promoter, because mouse polymerase cannot interact with the human promoter fragment (indented). Upon addition of human TAFs (expressed independently from constitutively active, inducible or tissue-specific promoters), polymerase I is able to achieve transcription.
**Figure 3:** Schematic representation of the experimental design of Tet-regulatable TAF-expressing vector. rtTA is expressed from a pol II promoter (can be tissue or cell type-specific). Upon addition of doxycycline, the drug will bind to rtTA. The complex will bind to the TRE of the response construct, leading to the induced expression of the human TAF, which in turn will then induce the pol I-driven expression of shRNA (not shown in Figure).
Figure 4: Specific suppression of Firefly luciferase activity by polymerase I-driven expression of short hairpin RNA targeting Firefly luciferase.
**Figure 5:** Activation of human polymerase I promoter by co-transfection of human TAFs in mouse hippocampal cell line.

The examples illustrate the invention.

### Example 1: Generation of a pol I polymerase-dependent system for suppressing Firefly luciferase expression in a hippocampal cell line.

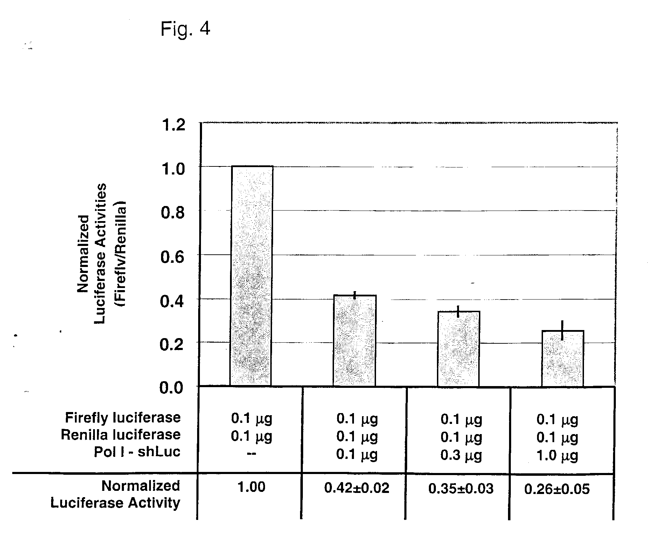

We cloned an inverted repeat (with a seperating core sequence of 8 nucleotides in length) into a modified mouse polymerase l-driven expression vector (kind gift from Prof. Grummt, DKFZ Heidelberg). The sequence is complementary to Firefly luciferase (29 bases in length) and will form a hairpin structure after transcription (as in Fig. 2). After processing, the short interfering RNA (siRNA) will be present for the degradation of mRNA coding for Firefly luciferase. The sequence is the same as published by Paddison et al. (Genes & Dev. 2002; 16: 948). The clone was termed Pol I-shLuc. This construct Pol I-shLuc was co-transfected in the mouse hippocampal cell line HT22 together with a vector expressing Firefly luciferase (the target of shLuc) and a vector expressing Renilla luciferase, which serves as an internal reference to normalize transfection efficiency. In fact, the Poll-shLuc construct was able to specifically suppress the activity of Firefly luciferase in a dose-dependent manner (Fig. 4). The efficiency of suppression is comparable with the polymerase III-driven vector as published by Paddison and co-workers.

### Example 2: Construction of a hybrid human-mouse polymerase I-driven expression vector

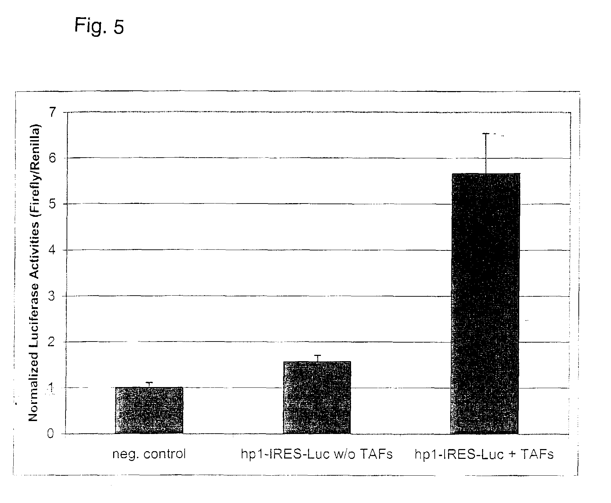

In addition, we constructed a hybrid human-mouse polymerase I-driven expression vector, containing functional parts of human promoter and mouse terminator sequences, with multi-cloning site between the two. We cloned the luciferase expressing DNA into the multi-cloning site, preceded by an internal ribosome entry site (IRES). The presence of IRES allows luciferase protein to be translated by ribosomes in the absence of typical mRNA configuration. The construct was termed hpl-IRES-Luc. The construct was transfected into mouse hippocampal cell line HT22 either in the absence or with the addition of three human TAFs (Fig.5). The luciferase expression without TAFs complementation was not different from mock-transfected cells. However, the addition of TAFs was able to enhance luciferase expression up to 5-6 fold as compared to negative control.

### REFERENCES

Brummelkamp T.R., Bernards R., and Agami R. (2002) A System for Stable Expression of Short Interfering RNAs in Mammalian Cells. *Science* 296, 550-553.
Elbashir S.M., Martinez J., Patkaniowska A., Lendeckel W., and Tuschl T. (2001) Functional anatomy of siRNAs for mediating efficient RNAi in Drosophila melanogaster embryo lysate. *EMBO J20,* 6877-6888.
Kwan K.M. (2002) Conditional alleles in mice: Practical considerations for tissue- specific knockouts. *Genesis.* 32, 49-62.
Lee R.C. and Ambros V. (2001) An extensive class of small RNAs in Caenorhabditis elegans. *Science* 294, 862-864.
Malleret G., Haditsch U., Genoux D., Jones M.W., Bliss T.P., Vanhoose A.M., Weitlauf C., Kandel E.R., Winder D.G., and Mansuy I.M. (2001) Inducible and reversible enhancement of learning, memory, and long-term potentiation by genetic inhibition of calcineurin. Cell 104, 675-686.
Miyagishi M. and Taira K. (2002) U6 promoter driven siRNAs with four uridine 3' overhangs efficiently suppress targeted gene expression in mammalian cells. *Nat. Biotechnol.* 20, 497-500.
Ohkawa J. and Taira K. (2000) Control of the functional activity of an antisense RNA by a tetracycline-responsive derivative of the human U6 snRNA promoter. *Hum. Gene Ther.* 11, 577-585.
Paddison P.J., Caudy A.A., Bernstein E., Hannon G.J., and Conklin D.S. (2002) Short hairpin RNAs (shRNAs) induce sequence-specific silencing in mammalian cells. *Genes Dev.* 16, 948-958.
Paul C.P., Good P.D., Winer I., and Engelke D.R. (2002) Effective expression of small interfering RNA in human cells. *Nat. Biotechnol.* 20, 505-508.
Rajewsky K., Gu H., Kühn R., Betz U.A.K., Müller W., Roes J., and Schwenk F. (1996) Perspective Series: Molecular Medicine in Genetically Engineered Animals. *J Clin Invest* 98, 600-603.
Yu J.Y., DeRuiter S.L., and Turner D.L. (2002) RNA interference by expression of short-interfering RNAs and hairpin RNAs in mammalian cells. *Proc. Natl. Acad. Sci. U. S. A* 99, 6047-6052.

## Claims

1. A combination of expression cassettes for interfering with the expression of at least one gene in a cell or a vertebrate comprising
(a) an expression cassette comprising a first promoter and operatively linked thereto a polynucleotide encoding an inducible transactivator (ITA);
(b) one or more expression cassettes comprising (a) polynucleotide(s) encoding one or more trans-acting factors (TAFs) heterologous to said cell or vertebrate and necessary and sufficient to functionally interact with a pol I promoter wherein expression of at least one of said TAFs is directed by a second promoter inducible by the transactivator; and
(c) at least one expression cassette comprising at least one pol I promoter and operatively linked thereto one or more polynucleotides giving rise upon transcription and optionally post-transcriptional modifications to RNA molecules capable of interfering with gene expression.

2. The combination of expression cassettes of claim 1 wherein said RNA molecules are short interfering RNAs (siRNAs) having a region of identity of 19 to 29 nucleotides of at least 90% with the exonic sequences of at least one target gene, ribozymes, antisense RNA oligonucleotides, RNA decoys, triplex-forming RNA oligonucleotides or RNA aptamers.

3. The combination of expression cassettes of claim 1 or 2 wherein said promoter inducible by said transactivator is a pol II polymerase promoter.

4. The combination of expression cassettes of claim 1 or 2 wherein said first promoter is a pol II polymerase promoter and is cell-type specific.

5. The combination of expression cassettes of any one of claims 1 to 4 wherein said vertebrate is a mammal.

6. The combination of expression cassettes of claim 5 wherein said mammal is a rodent.

7. The combination of expression cassettes of any one of claims 1 to 6 wherein said pol I polymerase promoter is a human pol I polymerase promoter.

8. The combination of expression cassettes of any one of claims 1 to 7 wherein said transactivator is inducible by an antibiotic or hormone.

9. The combination of expression cassettes of claim 8 wherein said antibiotic or hormone is tetracycline, doxycycline, ecdysone, ponasterone A, RU486, tamoxifen or a glucocorticoid hormone.

10. The combination of expression cassettes of any one of claims 1 to 9 wherein said inducible transactivator is a tetracycline-dependent transactivator (tTA), a reverse tetracycline-dependent transactivator (rtTA), an ecdysone receptor, a gal4-PR progesterone receptor chimera, a gal4-ER estrogen receptor chimera or glucocorticoid receptor (GR).

11. The combination of expression cassettes of any one of claims 1 to 10 further comprising at least one further polynucleotide molecule comprising a pol III promoter-driven expression cassette giving rise upon transcription and optionally posttranscriptional modifications to siRNA molecules having a region of identity of 19 to 29 nucleotides of at least 90% with the coding region of another target gene, or to ribozymes, antisense RNA oligonucleotides, RNA decoys, triplex-forming RNA oligonucleotides or RNA aptamers interfering with the expression of another target gene.

12. The combination of expression cassettes according to any one of claims 1 to 11 wherein the TAFs are TBP (TATA-binding protein), and TATA-associated factors, preferably TAFₗ48, TAFₗ63 and/or TAFₗ110.

13. A method of interfering with the expression of at least one target gene in a cell or a vertebrate comprising the step of providing said cell or said vertebrate carrying the combination of expression cassettes of any of claims 1 to 11, with an inducer of said transactivator.

14. A non-human transgenic animal harbouring the combination of expression cassettes according to any one of claims 1 to 11.

15. A cell line harbouring the combination of expression cassettes according to any one of claims 1 to 11.

16. A kit of parts comprising the combination of expression cassettes of any one of claims 1 to 11.
